Europäisches Patentamt

**European Patent Office**

**Office européen des brevets**

(11) Publication number: **0 444 675 A2**

# EUROPEAN PATENT APPLICATION

(21) Application number: **91103019.5**

(22) Date of filing: **28.02.91**

(51) Int. Cl.5: **G01N 21/47**, G01N 33/12, G01N 21/35

(30) Priority: **28.02.90 DK 534/90**

(43) Date of publication of application:
**04.09.91 Bulletin 91/36**

(84) Designated Contracting States:
**AT BE CH DE DK ES FR GB GR IT LI LU NL SE**

(71) Applicant: **Slagteriernes Forskningsinstitut**
**Maglegaardsvej 2**
**DK-4000 Roskilde(DK)**

(72) Inventor: **Borggaard, Claus**
**Birkedevejen 20**
**DK-4130 Viby Sjaelland(DK)**
Inventor: **Rasmussen, Allan J.**
**Baekgaarden 16**
**DK-2620 Albertslund(DK)**

(74) Representative: **Patentanwälte Beetz sen. -**
**Beetz jun. Timpe - Siegfried -**
**Schmitt-Fumian**
**Steinsdorfstrasse 10**
**W-8000 München 22(DE)**

(54) **Method and apparatus for determining the quality properties of individual pieces of meat.**

(57) The invention relates to a method and an apparatus for the determination of quality properties of individual pieces of meat, e.g. the water-holding capacity or fat marbling, by measuring the intensity of light reflected from the meat surface along a scanning line, to establish a histogram-like set of data relating to the frequency of reflection intensity values, and to calculate one or more characteristic values corresponding to a quality property with use of a suitable mathematical relation, the parameters of which are previously determined.

This concept allows a highly accurate and quick determination of meat quality properties and is particularly suited for on-line production and processing of meat.

EP 0 444 675 A2

The present invention relates to a method and an apparatus for determining quality properties of individual pieces of meat on the basis of the light reflection properties, wherein a measuring instrument provided with a reflection measuring device, e.g. an optical probe, is inserted into the meat, and the reflection intensity is measured along the insertion line, as defined in claims 1 and 11.

Today the meat industry delivers pork meat of good quality at very reasonable prices. This is due to comprehensive breeding work, rationalization at the pig producers, and quality control and high degree of efficiency at the slaughterhouses.

The average content of meat in pig carcasses has for instance been increased in Denmark after the introduction of a measuring system for determining the percentage of meat in the individual carcasses. Thus, the pig producers are paid in accordance with the meat content measured. This means that the meat cuts delivered to the consumers generally contain less fat.

The slaughterhouses aim at offering meat cuts which meet the special whishes of the consumers, e.g. meat which meets certain specifications as to colour, eating properties or fat-marbling. It is very difficult, however, to give any guarantee that such specifications have been complied with, because of the uncertainties connected with the methods available for the determination of quality properties of meat.

A simple classification of meat cuts into different quality classes may be made by an operator, but it would be extremely difficult to work out standards which are understood and complied with in the same way by all operators. It would also be highly time-consuming to make such a classification, particularly because the operators should receive a special training, and the training state should be kept up to date. Even so, the classification would be rather uncertain, since the operator can only assess a few external parameters of the meat.

Various measuring instruments for determining the optical properties of meat are known (GB-A-20 49 177, US-A-44 39 037, EP-A-28 509). DK-C-120 003 contains a description of a measuring instrument which comprises a probe with a light reflection meter in the form of a light emitter and a light detector. The probe is inserted into the meat, and the intensity of reflected light from the meat may then be read on a pointer instrument connected to the light detector.

These instruments are not very suitable for the classification of meat which is being processed at slaughterhouses or in the meat industry, as they are too inaccurate, among other things because the reading of the instruments would inevitably be affected by temperature fluctuations, wear and tear, and ageing of the optical components.

Even though the reflective power of the meat is an essential parameter, it is not necessarily the most accurate parameter for the determination of a quality property. The so-called PSE ('pale, soft, exsudative') meat which has inferior properties as roast or chops, is light meat, but this does not mean that all light meat is PSE meat. The content of pigment may be low in the meat in question, or the meat may for instance be fat-marbled and contain intramuscular fat. This is normally considered to give the meat extra good eating properties when used for roasts or chops.

An objective classification of meat, based upon the reflective power measured by means of the known instruments is thus inapplicable in practice. First of all because it results in a very high percentage of meat being sorted out, which is unacceptable for price and resource reaons. Secondly, the approved cuts of meat would have no fat-marbling which is an essential quality property.

There are various other known methods for the determination of certain quality properties in meat. It is well-known for instance that there is a correlation between PSE and the pH value of a piece of meat. This method cannot be utilized, however, under slaughterhouse conditions, as it takes a relatively long time for the measurable pH value to develop and stabilize. Moreover, the measurement is not very accurate.

There is a good correlation between PSE and the quantity of soluble protein in a meat piece. A normal or high content of soluble protein provides the meat a good water-retaining capacity. The methods known for the determination of soluble protein are based upon sampling and chemical analysis, which is highly demanding, technically, economically and as to work, if all pieces of meat on a slaughter line or a meat processing line are to be examined.

Thus, there is a need for providing a method and an apparatus applicable on production lines for determining quality properties of individual pieces of meat, e.g. for the detection of PSE meat. The meat may then be used for a production in which the water-holding capacity is of no importance for the eating properties, whereas the non-PSE meat may be supplied to the customers at normal prices on the whole, but with a guarantee for a juicy roast.

It is the object of the present invention to provide a method and an apparatus for the objective determination of quality properties of individual pieces of meat, especially for the detection of pieces of meat with deviant properties, such as PSE meat, so that the individual pieces of meat may be processed and sold in accordance with their properties. It should be possible to execute the method within a short period of time and with good accuracy on the production line in slaughterhouses and in the meat processing industry. Preferably, the method and apparatus should allow a determination of several different quality properties.

The above object is achieved according to the claims. The dependent claims relate to preferred embodiments.

The method of the present invention for determining quality properties of individual pieces of meat is based on

inserting an optical reflection measuring device into the meat and measuring the intensity of light reflected at the meat interface at a plurality of points along a scanning line determined by the insertion line of the measuring device, using light of a predetermined discrete wavelength or within a predetermined wavelength range, and eventually also measuring the insertion depth, wherein a set of measured values of the intensity of reflected light, is obtained, eventually in correlation with the insertion depth; it is characterized by the following essential steps:

(I)

- Calculating the numbers of occurences of measured values ($F_1$, $F_2$, ... $F_n$) falling within predetermined intervals covering the whole range or one or more partial ranges of possible measured values, for each measured value, eventually after conversion of the measured values into integers, and eventually after normalization of the measured values with respect to the irradiated light intensity,

or

- converting the measured values into integers, eventually with normalization with respect to the irradiated light intensity before or after that conversion, and calculating the numbers of occurences ($F_1$, $F_2$, ... $F_n$) of each of these integers,

to obtain a set of frequency data,

and

(II) calculating a value C characteristic for the respective quality property on the basis of a predetermined mathematical relation with use of the frequency data as variables.

The optical reflection measuring device may be a known optical probe according to the above-cited prior art.

The scanning line results from the insertion of the probe into the meat, i.e. the passage of the optical window of the probe through the meat.

In accordance with an alternative embodiment, the scanning line may be situated on the surface of a piece of meat. This method comprises the following steps:

(1) Illuminating a free or open surface of a piece of meat,

(2) measuring the intensity of light reflected at a meat surface at a plurality of points along one or more scanning lines by means of a scanner system, preferably a video camera, determining the scanning lines, using light of a predetermined discrete wavelength or within a predetermined wavelength range, and eventually also measuring or determining the distances of the measuring points from a reference point,

to obtain a set of measured values of the intensity of reflected light, eventually in correlation with the distances of the measuring points from the reference point,

and

(3) determining the quality property by

(I)

- calculating the numbers of occurences of measured values ($F_1$, $F_2$, ... $F_n$) falling within predetermined intervals covering the whole range or one or more partial ranges of possible measured values, for each measured value, eventually after conversion of the measured values into integers, and eventually after normalization of the measured values with respect to the irradiated light intensity,

or

- converting the measured values into integers, eventually with normalization with respect to the irradiated light intensity before or after that conversion, and calculating the numbers of occurences ($F_1$, $F_2$, ... $F_n$) of each of these integers,

to obtain a set of frequency data,

and

(II) calculating a value C ($C_{whc}$, $C_{fat}$) characteristic for the respective quality property on the basis of a predetermined mathematical relation with use of the frequency data as variables.

This second method of the present invention has the particular advantages that there is no necessity of inserting a probe into the piece of meat, and a large number of scanning lines can be measured very quickly and with high accuracy.

The processing of the measured values of the reflected light intensity may be carried out by means of devices comprising a hardwired program, but preferably with use of computer systems and particularly microprocessor systems.

The respective input data corresponding to measured values are A/D converted, if necessary, and processed in accordance with predetermined programs.

According to another preferred embodiment, the computer system is a learning system regarding the determination of the mathematical relation used for the calculation of the respective quality property value and/or the determination of coefficients, or constants based on reference values obtained by means of other methods.

In accordance with a preferred embodiment, the apparatus of the present invention for carrying out the above-described method comprises

(A1) an optical reflection measuring device which can be inserted into the meat and measures the intensity of light reflected at the meat interface at a plurality of points along a scanning line determined by the insertion line of the measuring device and which eventually also measures the insertion depth, or

(A2) illuminating means for reproducibly and constantly illuminating the surface of a piece of meat and a scanner system, preferably a video camera system, measuring the intensity of light reflected at the meat surface at a plurality of points along one or more scanning lines with light of a predetermined discrete wavelength or within a predetermined wavelength range,

(B) a processing unit comprising an I/O device, an A/D-converter and an arithmetic unit including a CPU, a ROM and a RAM, and being designed to carry out the following steps:

(I)

- calculating the numbers of occurences of measured values ($F_1$, $F_2$, ... $F_n$) falling within predetermined intervals covering the whole range or one or more partial ranges of possible measured values, for each measured value, eventually after A/D conversion of the measured values, and eventually after normalization of the measured values with respect to the irradiated light intensity,

or

- A/D converting the measured values, eventually with normalization with respect to the irradiated light intensity before or after that conversion, and calculating the numbers of occurences ($F_1$, $F_2$, ... $F_n$) of each of these values,

to obtain a set of frequency data, eventually with determination of the distances of the measuring points from a reference point and correlation of the measured values with these distances,

and

(II) calculating a value C ($C_{whc}$, $C_{fat}$) characteristic for the respective quality property on the basis of a predetermined mathematical relation with use of the frequency data as variables,

and

(C) output means for displaying, storing, transferring, transmitting, printing and/or plotting the determined characteristic quality values, and/or for controlling a transport route for examined pieces of meat and/or actuating a marking device therefor.

The normalization of the measured reflected light intensity, $I_{refl}$, with respect to the intensity of irradiated light, $I_o$, is preferably carried out on the basis of the ratio $I_{refl}/I_o$. Further, the total range of possible reflected light intensities or normalized values may be subdivided into intervals, preferably of equal width, for establishing the histogram data according to step I.

The mathematical relation used in step II for calculating the quality property may be any suitable relation.

In accordance with another preferred embodiment of the method of the present invention, the characteristic value C is determined from a nth order polynom of the formula

$$C = K_o + K_1 \cdot F_1 + K_2 \cdot F_2 + ..... K_n \cdot F_n ,$$

wherein $K_o$ and $K_1$ to $K_n$ are predetermined constants, and $F_1$ to $F_n$ are the frequency data corresponding to the numbers of measured values, the constants $K_o$ and $K_1$ to $K_n$ preferably being determined on the basis of frequency data ($F_1$, $F_2$, ... $F_n$) and reference values relating to the respective quality property C.

This mathematical relation corresponds to an algorithm which, on the other hand, is comprised in the software of the preferred apparatus of the present invention.

4

By means of the present method it has, surprisingly, proved possible to determine for instance the water-holding capacity ('whc' ) of individual pieces of meat with an accuracy which is so good that it has become possible in practice to detect PSE meat on-line. Such a detection has not been possible so far, not even by time-consuming and costly analyses.

In order to execute the method according to the invention it is only necessary to insert an optical probe of a measuring instrument in the piece of meat and to process the obtained values. The method may be carried out very quickly without sampling or destruction of the meat.

The concept of the invention is highly tolerant to inaccuracies of measurements which occur in the type of measuring instruments used and which are due to the highly fluctuating working conditions as well as wear and tear and ageing of the optical components.

An advantage of the method and apparatus according to the invention is that several quality properties may be determined by the same measuring operation. All that is needed is an instrument comprising different data processing configurations corresponding to the types of quality properties looked for. It has proved possible for instance to quantitatively determine both the water-holding capacity and the fat-marbling ('fat') on the basis of reflection values obtained by a single scanning measurement of the meat piece. That is of very great interest if the slaughterhouse wants to supply e.g. cuts of loin which are PSE free and which also have a certain fat-marbling. In this way it is possible to guarantee the customers roasts of good eating quality.

If a measuring instrument provided with a detector for determining the depth of insertion of the probe into the piece of meat for each measurement is used, it is also possible to determine the thicknesses of meat and/or fat of the piece of meat in the same measuring operation as the one for determining the quality properties according to the method of the invention. These thicknesses may be of importance for the assessment as to whether the piece of meat is suitable for a specific use.

Thus, the probe measurement and the consequent processing of the reflection values will show which of several different productions the meat piece is most suited for.

In order to get a good basis for the determination of the quality properties of a piece of meat it is advisable to make many measurements along the scanning line, as the reflective power of a piece of meat may vary because of its individual muscle structure. According to one embodiment, measurings of the light-reflective power are made at not less than 10 different points along the scanning line. In practice, 100 - 200 measurements may be made in the meat per insertion.

The measurements should be distributed over the entire length of the scanning line which is located in the actual meat section of the piece of meat, but it is also possible to restrict the measuring operations to a smaller section of the piece of meat, e.g. the muscular section where a deviant meat quality is known to occur, such as PSE.

If desired, a probe measuring instrument may be applied in which the light-reflection meter is combined with a distance-measuring device which measures the depth of insertion of the probe into the piece of meat; the device releases a reflection measurement for each little step the probe is inserted further into the meat piece. The light-reflective power may be measured for at least every 10 mm on the scanning line. In practice, it is possible to make a measurement for e.g. every 0.25 mm.

The reflection measurements from fat and air which the probe passes through during the insertion or the extraction may be distinguished from the meat reflection measurements by analysing the curve showing the reflection values as a function of the depth of insertion. Fat has a high reflective power and air a very low reflective power, whereas the reflection values for meat are somewhere in between.

Due to the red colour of the meat the measurement of the light reflective power is primarily made in the red region of the spectrum, on the transition between the visible and the near-infra-red spectrum, especially at 950 nm, as this gives reflection values of average height, but the measurements may also be made in other regions or over the entire visible region or the invisible region, such as the NIR or the UV region.

In measuring instruments of the type in question, the reflection value is usually obtained as an integer, as the signal of the probe is converted into an integer by means of an A/D-converter. If the values are applied as such, there is no need for any further processing for the computer unit to decide how many times the individual values occur or are repeated. In order to reduce the noise or simplify the algorithmic calculation, however, strongly deviant reflection values may be filtered away prior to the decision mentioned, so that the remaining reflection values are typical for the reflection from muscles. A measuring range of 0 - 200 light reflection units may for instance be narrowed to the range of 30 - 150 units which substantially covers the reflection values from muscles.

It may be advantageous to simplify the calculation of the number of times the individual values occur or are repeated by combining several of the possible neighbouring values. In this case it is part of the calculation to find out how many of the measurements will fall within the connected intervals formed. Each

5

interval may e.g. comprise five consecutive, possible integer values. In this way the calculation according to the algorithm would also be more simple, as it would comprise a correspondingly lower number of variables.

Thus, the values obtained by the scanning operation may be arranged within intervals, each interval comprising e.g. five units of reflection intensity or reflection power, and all values within an interval may be regarded as having the same integer value.

Before the calculation in question is made in the computer unit, the reflection values obtained may also be processed for eliminating offset errors or e.g. amplifying or attenuating fluctuations of the values.

For each reflection measurement a value may be formed which is attenuated or smoothed out with respect to the neighbouring measurements, and the difference between the original reflection values and the attenuated values may be used as results of measurement in the form of integers.

The probe instrument applied may be designed to measure within different wavelength ranges or of different discrete wavelengths. In this way it is possible to improve the accuracy or to determine different quality properties of meat by means of the same instrument, wherein the correlation between the light reflection and the properties is to be established at two or more different wavelengths or within two or more wavelength regions.

Furthermore, it is possible to determine various other quality properties of meat by recording a spectrum of the reflection of the meat, data processing the reflection values in the individual bands and processing the achieved values according to an algorithm which expresses the quality properties, e.g. the content of pigment. By applying one wavelength or changing wavelengths it is possible to apply the method which is the most optimal for the determination in question.

Thus, the probe applied may be a probe which can measure in different wavelength regions or at different discrete wavelengths, and different quality properties may be measured on the basis of the light-reflection properties in different wavelength regions or at discrete wavelenghts.

The multi-variable mathematical relation or algorithm applied has a number of variables corresponding to the number of integers or intervals which, according to a decision, are to form the basis of the determination. The number and the width of the intervals are ruled by the conditions in practice, e.g. the desired accuracy. The accuracy would generally improve with increasing number of variables. There may e.g. be 10 variables if the significant values measured are between 50 and 99 reflection units, and this range of measurement is divided into 10 intervals, each comprising five units.

The algorithms applied preferably comprise 5 to 50 variables.

There are mathematical models and the respective software available which allow the development of an algorithm which has many variables and which is suitable for the purpose described. They are known under the designation of multi-variable calibration models.

The algorithm may be provided by multi-variable calibration by means of a set of calibration data consisting of reflection values measured in pieces of meat and corresponding reference values expressing the property.

The reflection values at various points along the scanning line are determined in a number of samples, e.g. 50 samples representing a good span. Then the desired property, e.g. the water-holding capacity, is determined of each of the samples, by means of a reliable, per se well-known method of analysis. The data obtained may easily be processed in a computer program. The program calculates the constants in the applied type of multi-variable algorithm as the values which in toto give the best agreement between the property values calculated by means of the algorithm and the reference values.

By feeding reference values for another property of the samples into the program, e.g. fat-marbling, the program may calculate, on the basis of the same reflection values. Another property of pieces of meat may be determined in accordance with another algorithm.

A special mathematical method that is applied in connection with multi-variable calibration is called the partial least squares method. It makes it possible to detect, among other things, whether a sample or a determination is outside what is known from the samples used for the calibration.

Another method is the principal components regression method. Thus, the mathematical relation or algorithm may be determined by the partial least squares method or the principal components regression method.

The method according to the invention may be applied to determine any quality property which shows a correlation with the reflective power of meat. Essential meat quality parameters which may for instance be determined are the water-holding capacity (PSE) and the fat-marbling.

The present method may also be applied in connection with measurements of reflections made on a free or open surface of meat by means of e.g. a scanner system without an insertion probe, such as a video camera.

6

The present method may be applied to meat, such as red meat from pigs, bulls, young steers, cows, calves, deer, sheep, lambs, etc.

The present method may e.g. be executed by means of instruments comprising a probe for the measurement of the reflective power of meat, and a data processing unit designed to release a series of measurements and receive the values measured during the insertion of the probe into the meat. The processing unit stores a multi-variable algorithm. The unit should be designed to input the achieved integers, to make a calculation therefrom according to the algorithm and to output the result of the determination via any output channel.

By means of this apparatus it is possible to determine very quickly and accurately, e.g. the water-holding capacity of individual pieces of pork. Thus, it is possible to establish whether a piece of meat for instance is PSE, which has not been possible with the optical instruments known so far. The apparatus may perform the determination on-line on a slaughter or production line, and the result may be presented so quickly that the piece of meat may be graded or marked immediately in accordance with the result.

The output channel may comprise a display with information for the operator. It may also comprise a control box which has been designed to control the transport route for an examined piece of meat and/or to actuate a marking device for the piece of meat.

By means of the method and apparatus according to the invention it is possible to grade pieces of meat depending on their properties fully automatically or semi-automatically.

The invention also relates to a method for the detection of pieces of meat with pre-determined quality properties. The method comprises that the quality property value or values of the individual pieces of meat are determined by means of the method according to Claims 1 or 2, and that the values are compared with pre-determined threshold limit values, after which the pieces of meat, on the basis of comparison result, are assigned to a quality group.

The method makes it possible to provide the customers with cuts of meat that comply with certain quality requirements, such as very juicy roasts with fine eating qualities.

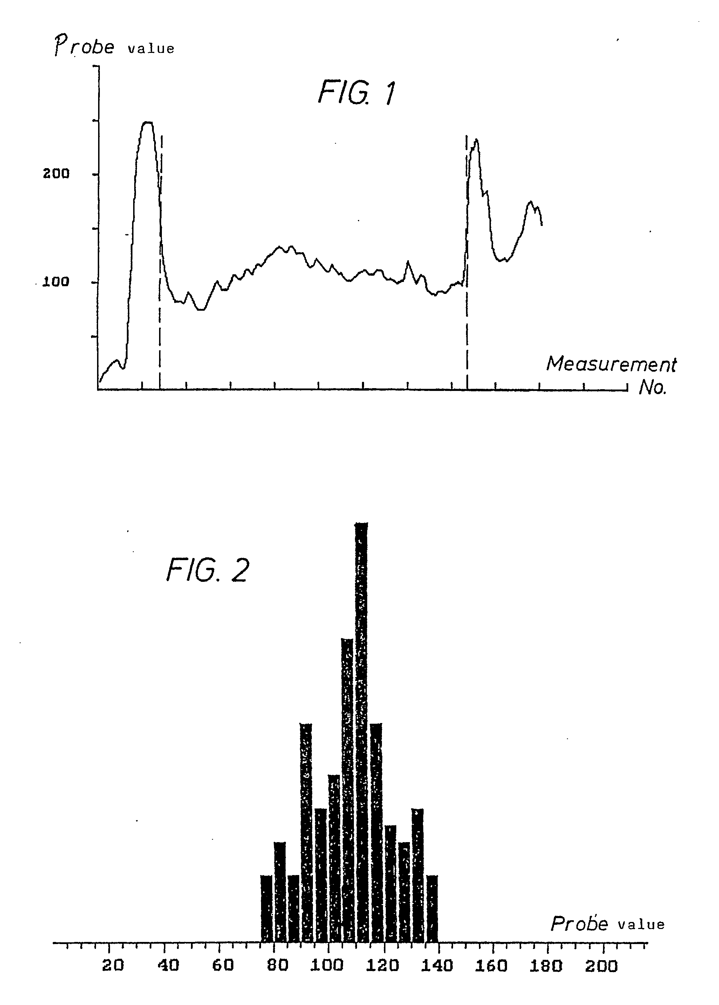

The invention is described in further detail in the following Examples with reference to Figs.1 to 8. The figures stated in the Examples and Figures as representing probe values, lab. whc, pred. whc, lab. fat and pred. fat are in arbitrary units.

## EXAMPLE 1

From a large quantity of loins a number of 110 loins is selected, representing a wide variety of meat qualities, right from very light meat to dark meat, and from no fat-marbling to heavy fat-marbling. This selection of loins is not representative of the meat usually occurring in the production, as the objective is to get a uniform number of each quality. A series of measurements of the reflective power of these loins are made by means of a measuring pistol provided with a probe with a reflection meter.

The probe is inserted into the loin, and a number of reflection values are recorded while the probe passes through the piece of meat. The measuring pistol may include a depth-measuring device which registers the depth of insertion of the probe and which automatically releases a measuring cycle for every 0.5 mm the probe is inserted further into the meat. The reflection values are converted by means of an A/D-converter into digital integers which, via a conductor, are transferred to a computer unit for storage. In this way, a reflection profile is established for each insertion into the individual loins, as illustrated in Fig. 1.

The peaks of the curve to the left and to the right in the Figure correspond to the fat layer of the loin, whereas the curve between these peaks represents the meat range, the qualities of which are to be determined by the measurement. It may be seen that the reflective power of the meat, in the case illustrated, varies somewhat through the muscle, which may be due, among other things, to PSE-spots or intramuscular fat. After the insertion, the curve is shown on a display unit to the operator, who will approve the measurement. Two different probe meters are used for the measuring. Two insertions are made into the loins with each probe meter at pre-determined places.

Immediately after the probe measurement has been made, a sample is taken around the insertion spot, and this sample is minced on the spot and transported to the laboratory for analytical determination of the content of soluble protein (water-holding capacity).

### Data processing

The series of reflection values stored in the computer unit is then analysed by means of a programme determining where the values start representing reflections in meat, and where the values no longer represent reflections from meat. In this way the meat range of the profile is determined, as indicated by the dotted lines in Fig. 1. The achieved collection of data is consequently freed from data concerning reflections from fat and air.

The number of meat reflection values registered by such a measuring operation depends on the thickness of the muscle, since the number of reflection values recorded increases with the thickness of the meat part. The computer unit compensates for the varying number of reflection values by normalizing the amount of data. The original reflection values are processed so as to establish e.g. 100 reflection values for each insertion. The normalization may be made by selection, when there are more than 100 original reflection values, or by establishing new, artificial reflection values made as an average of two neighbouring values, when there are fewer than 100 original reflection values, or by another known mathematical model for the normalization of a data set.

Then, a programme in the computer unit counts how frequently the different reflection values occur in the data collection. By way of illustration, Fig. 2 shows a histogram of the frequency of the values measured. As may be seen, the muscle in question is rather heterogeneous, the reflection values varying from probe value 75 to probe number 140.

## Water-holding capacity - loins

An algorithm for the water-holding capacity is stored in the computer unit. The algorithm has the form

$$C_{whc} = K_0 + K_1 \cdot F_1 + K_2 \cdot F_2 + \ldots\ldots K_n \ F_n$$

in which $C_{whc}$ is the water-holding capacity of the meat, $K_0$ to $K_n$ are pre-calculated constants, and $F_1$ is the number of actual values with reflection value 1, $F_2$ is the number of values with reflection value 2, etc.

Reflection value No. 1 may e.g. correspond to a measured reflection value of 70, whereas the value n correponds to a measured reflection value of 150, so that the algorithm has 81 variables. Several measured reflection values may be combined to simplify the algorithm. Reflection value No. 1 may e.g. correspond to measured reflection values from 70-74, whereas value n corresponds to measured values from 150-154. The algorithm will then have 17 variables.

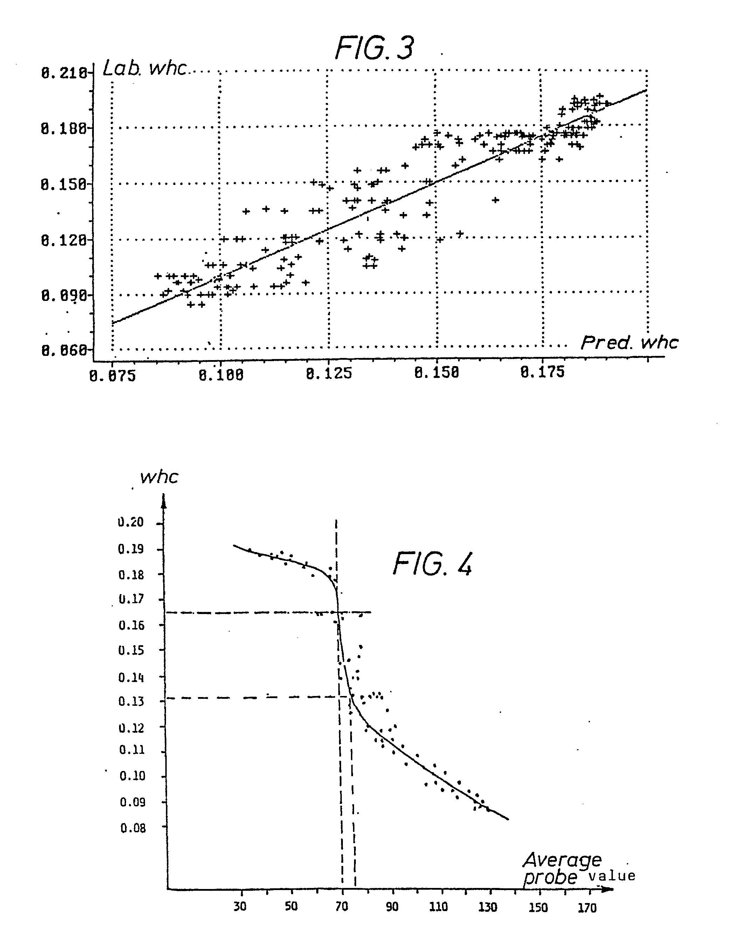

For each of the approved measurements a calculation is now made of the water-holding capacity, the numbers found by the above processing of the series of reflection measurements being inserted into the algorithm, and $C_{whc}$ is calculated by the computer programme. The results, based upon the 319 approved measurements, are illustrated together with the analytically determined water-holding capacity in Fig. 3. A correlation coefficient of 0.93 is calculated, which means that there is a good correlation between the analytically determined (Lab. whc) and the optically determined water-holding capacity (Pred. whc). The present optical determination by probe insertion and processing of the achieved series of reflection values may consequently be applied with good certainty for quick, objective detection of meat with normal water-holding capacity, e.g. more than 0.140.

Meat with a water-holding capacity below this limit is strongly over-represented in Fig. 1 in proportion to the production at slaughterhouses etc.

For these experiments,
it has been necessary, for practical reasons, to perform the reflection measurements under two different conditions, partly in a cold storage room on suspended carcasses, and partly on the cutting line on cut-up loins. In the cold storage room the measuring operations were on the whole made on loins with a water-holding capacity of > 0.16 and < 0.11, whereas loins with a water-holding capacity in the interval between the two limits were substantially measured on the cutting line, where they were easy to find.

Part of the variation in the interval 0.12-0.15 is due to the analytical method, since the meat was very heterogeneous, which makes it difficult to take a representative sample of meat.

## Construction of the Algorithm

Out of the above-mentioned 319 measurements made on loins, 101 were used as training sets for the construction of the algorithm.

After the data processing for isolation of the meat reflection values, normalization of each set of data, and registration of the frequencies of the data collection at the individual, possible light-reflection values, the frequencies found in each of the 101 data sets and the correspcnding values for water-holding capacity, found by the analytical method, are inserted into a programme which determines, by means of these data, the constants of a corresponding multi-variable algorithm of the type shown above, so that the best possible agreement in the training set is achieved between the water-holding capacity, which is calculated by means of the frequencies found, and the analytically determined values for the water-holding capacity.

The constants are put into the algorithm in the programme of the computer unit, after which the algorithm may be applied for the calculation of the water-holding capacity of a loin, based upon the probe reflection values, as shown above.

## Comparison

In order to examine the accuracy of the multi-variable algorithm as compared with the accuracy of a more traditional determination of the water-holding capacity, the following calculation was made. The average of the registered reflection values measured was calculated for a number of measurements obtained by insertion, and these values were plotted into a coordinate system together with the corresponding values obtained by analysis for the water-holding capacity, see Fig. 4.

Fig. 4 shows that there is no linear correlation between the average light-reflection value and the water-holding capacity. In the probe value region between 70 and 77 the curve falls sharply, and this may be the explanation why with probe measurements it is difficult to find loins with analysis values of between 0.125 and 0.165.

It is quite surprising that the present multi-variable method is so good for predicting the water-holding capacity in this region, since a change in the probe number of 1 might be expected to bring about a change in the water-holding capacity of 4.1%, as is the case with the average method. However, the multi-variable method permits a variation in the reflection value through the meat section, and therefore this method is more accurate than the average method.

The non-linearity between water-holding capacity and reflection values, together with the improved accuracy in the transitional region, turns out to be of great advantage in grading, where in a given production muscles with PSE are to be sorted out in a relatively accurate way, so that the customers get a guarantee of juiciness, but also so that the amount of meat with good juiciness, sorted out for a different production, is not too big.

## Probe calibration

It is common for optical probe instruments that the reflection meter gets out of calibration due to wear and tear, ageing of the optical components, and variation in the ambient temperatures. A measurement which shows values that are two or three probe values too high or low may mean, at the average value mentioned above, an error of 10% in the water-holding capacity. In order to examine whether the multi-variable algorithm is robust to a change in the calibration level of the probe meter, all reflection values of a measurement were multiplied by 1.04 and 0.96, respectively, and the obtained values were processed in the same way as mentioned above and put into the algorithm. The water-holding capacity was calculated and compared with the originally calculated water-holding capacity. The results appear from the Table below.

| Originally calculated water-holding capacity interval | Change in the calculated water-holding capacity at 1% decalibration |
|---|---|
| 0.080 - 0.089 | 0.0010 |
| 0.090 - 0.099 | 0.0010 |
| 0.100 - 0.109 | 0.0008 |
| 0.110 - 0.119 | 0.0007 |
| 0.120 - 0.129 | 0.0008 |
| 0.130 - 0.139 | 0.0014 |
| 0.140 - 0.149 | 0.0013 |
| 0.150 - 0.159 | 0.0014 |
| 0.160 - 0.169 | 0.0012 |
| 0.170 - 0.179 | 0.0008 |
| 0.180 - 0.189 | 0.0002 |

It may be seen that the change over the entire area of measurement, incl. the area 0.130 - 0.165, is fairly constant at 0.001 at 1% decalibration of the probe meter (corresponding to 1.5 probe value). Therefore, the present method is considerably more stable than the average method, where a decalibration of 1.5 probe value in the area mentioned would give a change in calculated water-holding capacity in the magnitude of 0.01.

This improvement in accuracy may be due to the fact that the algorithm in question is not too sensitive regarding the probe level as well as variations in the reflective power through the meat.

## Individual Probe

In optical probe meters differences may occur between individual instruments due to differences in the manufacture of the components and due to dislocations of components.

Measurements made in the same piece of meat with two different probes were processed in accordance with the present method, and the calculated water-holding capacities were compared. A correlation coefficient of 0.97 and an $R^2$-value of 0.94 were found, which shows that there is a very high agreement between the measurements, and a very big part of the uncertainty lies in the sample-taking technique applied in connection with the analysis. The optical measurement may therefore give a more accurate picture of the meat quality than the analytical method.

## EXAMPLE 2

### Water-holding capacity - Silversides

In the same way as described in Example 1, 252 measuring series are recorded of reflection values achieved by insertion of a probe into a number of silversides. The data are processed, and the figures obtained are put into a multi-variable algorithm which expresses the water-holding capacity. These measurements have not been used for determining the constants of the algorithm. Samples from the silversides used are analysed to determine the analytical water-holding capacity.

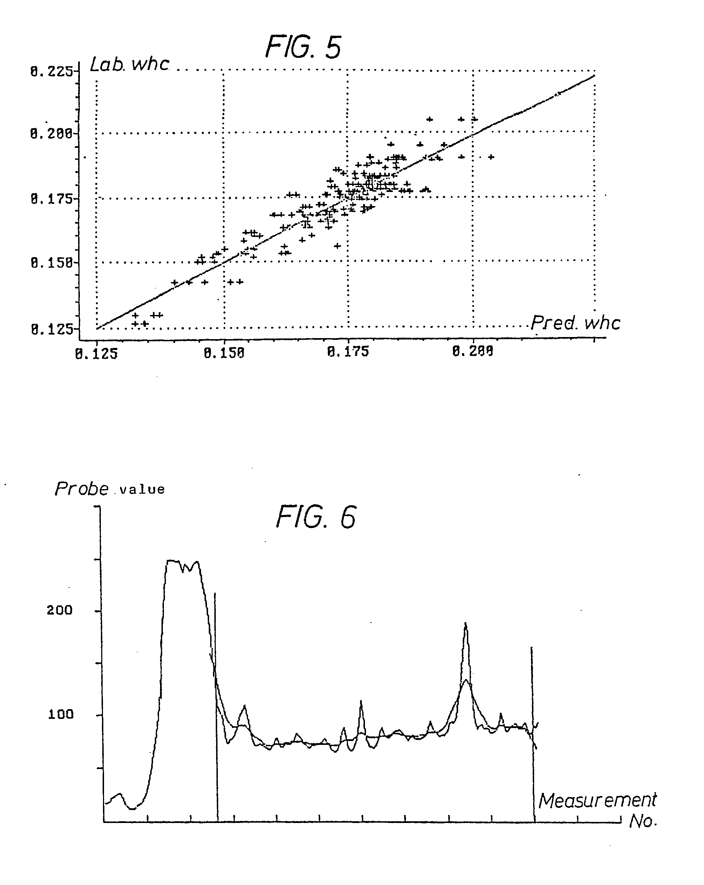

The result is illustrated in Fig. 5. A correlation coefficient of 0.90 is found. As may be seen, there is a much smaller span in the material than was the case for the loin measurements, which may be due to the meat selection method applied. There are very few data available for silversides whose water-holding capacity is below 0.130.

10

### Determination of Algorithm

The algorithm applied above was constructed in the same way as stated in Example 1. 84 measuring series were applied as training sets, recorded by insertion of an optical probe into different silversides while a series of reflection values were registered.

Samples of the silversides are analysed for their water-holding capacity and by means of a software programme in the computer unit the constants in the algorithm are found which show the best agreement between the water-holding capacities calcultated by means of the algorithm and the analytically determined water-holding capacities. For the final algorithm the correlation coefficient was found to be 0.92.

## EXAMPLE 3

### Fat-marbling

In this Example, the 53 loins used have a water-holding capacity which is higher than 0.15. The water-holding capacity was determined by analysing a sample in accordance with a laboratory method.

In the same way and with the same equipment as in Example 1, reflection profiles of loins are recorded. The probe of a measuring pistol is inserted into a loin, and a series of reflection values is recorded while the probe passes through the meat. The values are stored in the memory of the computer unit, and the values corresponding to the meat regions of the loins are isolated by means of the same data processing as the one in Example 1.

In the computer unit there is a software programme which by the processing of the stored reflection values forms a new set of data with smaller fluctuations in the reflection values. The software programme may e.g. for each measurement of reflection form a new value which is an average of the values found in the immediately preceding and following measurements, and this method may be repeated so that a further smoothing is achieved.

In this way one gets two sets of data, one set with the original values and one set with the smoothed-out values, corresponding to the curves illustrated in Fig. 6 of the original reflection profile and the smoothed-out profile.

In the computer unit a new set of data is formed, the individual elements of which are the differences between the original values and the smoothed-out values, i.e. corresponding to the integer differences between the two curves of Fig. 6.

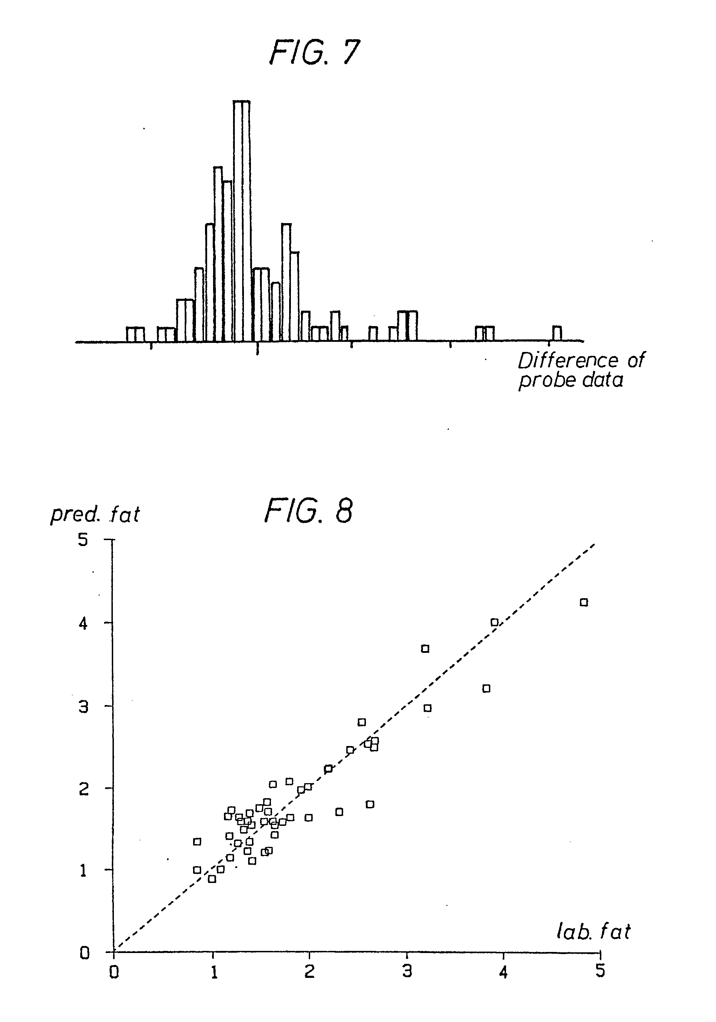

A normalization is made in the same way as described in Example 1, so that the number of difference values becomes 100. The frequency of each of the differences occurring is counted. Fig. 7 shows a histogram of differences achieved in this way.

A narrow curve in the histogram indicates that the loin in question has a low degree of fat-marbling, whereas a wide curve indicates that the meat is heavily fat-marbled.

The frequencies achieved in this way for each occurring difference are put into the following algorithm, which has been stored into the computer unit

$$C_{fat} = K_o + K_1 \cdot F_1 + K_2 \cdot F_2 + \ldots\ldots K_n \cdot F_n$$

in which $C_{fat}$ means the content of fat in the meat (i.e. degree of marbling), $K_o$ to $K_n$ are constants, and $F_1$ to $F_n$ are the found frequencies of the various differences between the original reflection values and the smoothed-out reflection values.

The results are shown in Fig. 8. The correlation coefficient between the analytically determined content of fat (lab. fat) and the optical determination of the fat-marbling (pred. fat) is 0.93.

### Determination of Algorithm

The constants of the above-mentioned algorithm were found on the basis of the same measurements as those above.

The set of data corresponding to the histogram, together with corresponding sets of data from the other measurements of loins, were used as training sets for the development of the model, which gives the best possible agreement between the analytically determined and the optically determined content of fat. The computer unit includes a programme which performs the necessary data processing and calculates the constants of the model. The so-called partial least squares method is applied in the programme.

The meat collection used was far from perfect for the development of the algorithm, as the meat was primarily selected to provide information about the water-holding capacity of meat. As may be seen, meat with a content of fat of more than 3% is very poorly represented.

## Claims

1. Method for determining quality properties of individual pieces of meat by

    inserting an optical reflection measuring device into the meat and measuring the intensity of light reflected at the meat interface at a plurality of points along a scanning line determined by the insertion line of the measuring device, using light of a predetermined discrete wavelength or within a predetermined wavelength range, and eventually also measuring the insertion depth, to obtain a set of measured values of the intensity of reflected light, eventually in correlation with the insertion depth,

    **characterized by**
    (I)
    - calculating the numbers of occurences of measured values ($F_1$, $F_2$, ... $F_n$) falling within predetermined intervals covering the whole range or one or more partial ranges of possible measured values, for each measured value, eventually after conversion of the measured values into integers, and eventually after normalization of the measured values with respect to the irradiated light intensity,

    or
    - converting the measured values into integers, eventually with normalization with respect to the irradiated light intensity before or after that conversion, and calculating the numbers of occurences ($F_1$, $F_2$, ... $F_n$) of each of these integers,
    to obtain a set of frequency data,

    and
    (II) calculating a value C ($C_{whc}$, $C_{fat}$) characteristic for the respective quality property on the basis of a predetermined mathematical relation with use of the frequency data as variables.

2. Method for determining quality properties of individual pieces of meat on the basis of the measured intensity of light reflected therefrom,

    characterized by the following steps:
    (1) Illuminating a free or open surface of a piece of meat,
    (2) measuring the intensity of light reflected at a meat surface at a plurality of points along one or more scanning lines by means of a scanner system, preferably a video camera, determining the scanning lines, using light of a predetermined discrete wavelength or within a predetermined wavelength range, and eventually also measuring or determining the distances of the measuring points from a reference point,

    to obtain a set of measured values of the intensity of reflected light, eventually in correlation with the distances of the measuring points from the reference point,

    and
    (3) determining the quality property by
    (I)
    - calculating the numbers of occurences of measured values ($F_1$, $F_2$, ... $F_n$) falling within predetermined intervals covering the whole range or one or more partial ranges of possible measured values, for each measured value, eventually after conversion of the measured values into integers, and eventually after normalization of the measured values with respect to the irradiated light intensity,

    or
    - converting the measured values into integers, eventually with normalization with respect to the irradiated light intensity before or after that conversion, and calculating the numbers of occurences ($F_1$, $F_2$, ... $F_n$) of each of these integers,

to obtain a set of frequency data,

and
(II) calculating a value C ($C_{whc}$, $C_{fat}$) characteristic for the respective quality property on the basis of a predetermined mathematical relation with use of the frequency data as variables.

3. The method according to claims 1 and 2, characterized in that the intensity of reflected light is measured at not less than 10 different points, preferably having equal distance.

4. The method according to claims 1 to 3, characterized in that the intensity of reflected light is measured after predetermined time intervals after insertion of the reflection measuring device.

5. The method according to claims 1 to 4, characterized in that the intensity of reflected light is measured at measuring points of constant, predetermined distance, preferably $\leq$ 10 mm.

6. The method according to claims 1 to 5, characterized in that the intensity of reflected light is measured at two or more different discrete wavelenghts or within two or more predetermined wavelength ranges, and several quality properties are determined on the basis of frequency data obtained for the different discrete wavelengths or the different wavelength ranges.

7. The method according to claims 1 to 6, characterized in that the intensity of reflected light is measured within the red region of the spectrum in the transition region between the visible and the near-infrared region, preferably at 950 nm.

8. The method according to claims 1 to 7, characterized in that the measured values are attenuated or smoothed-out with respect to neighbouring measured values, and the differences between the original measured values and the respective attenuated values are used as frequency data for the calculation in step II.

9. The method according to claims 1 to 8, characterized by determining one or more quality properties of the meat by calculating the respective characteristic values C ($C_{whc}$, $C_{fat}$), and comparing these values with predetermined threshold limit values corresponding to quality groups, and assigning the meat to the respective quality group in accordance with the comparison results.

10. The method according to claims 1 to 9, characterized in that the characteristic value C ($C_{whc}$, $C_{fat}$) is determined from a nth order polynom of the formula

$$C = K_o + K_1 \cdot F_1 + K_2 \cdot F_2 + .....K_n \cdot F_n ,$$

wherein $K_o$ and $K_1$ to $K_n$ are predetermined constants, and $F_1$ to $F_n$ are the frequency data corresponding to the numbers of measured values, the constants $K_o$ and $K_1$ to $K_n$ preferably being determined on the basis of frequency data ($F_1$, $F_2$, ... $F_n$) and reference values relating to the respective quality property (C).

11. The method according to claims 1 to 9, characterized in that the mathematical relation used in step II is established by means of a multi-variable calibration model, particularly the partial least squares method or the principal components regression method, based on frequency data ($F_1$, $F_2$, ... $F_n$) and reference values relating to the respective quality property (C).

12. An apparatus for determining quality properties of individual pieces of meat, particularly for carrying out the method of claims 1 to 11,

comprising
(A1) an optical reflection measuring device which can be inserted into the meat and measures the intensity of light reflected at the meat interface at a plurality of points along a scanning line determined by the insertion line of the measuring device and which eventually also measures the insertion depth,

or

(A2) illuminating means for reproducibly and constantly illuminating the surface of a piece of meat and a scanner system, preferably a video camera system, measuring the intensity of light reflected at the meat surface at a plurality of points along one or more scanning lines with light of a predetermined discrete wavelength or within a predetermined wavelength range,

(B) a processing unit comprising an I/O device, an A/D-converter and an arithmetic unit including a CPU, a ROM and a RAM, and being designed to carry out the following steps:

(I)

- calculating the numbers of occurences of measured values ($F_1$, $F_2$, ... $F_n$) falling within predetermined intervals covering the whole range or one or more partial ranges of possible measured values, for each measured value, eventually after A/D conversion of the measured values, and eventually after normalization of the measured values with respect to the irradiated light intensity,

or

- A/D converting the measured values, eventually with normalization with respect to the irradiated light intensity before or after that conversion, and calculating the numbers of occurences ($F_1$, $F_2$, ... $F_n$) of each of these values,

to obtain a set of frequency data, eventually with determination of the distances of the measuring points from a reference point and correlation of the measured values with these distances,

and

(II) calculating a value C ($C_{whc}$, $C_{fat}$) characteristic for the respective quality property on the basis of a predetermined mathematical relation with use of the frequency data as variables,

and

(C) output means for displaying, storing, transferring, transmitting, printing and/or plotting the determined characteristic quality values, and/or for controlling a transport route for examined pieces of meat and/or actuating a marking device therefor.

FIG. 1

FIG. 2

FIG. 3

FIG. 4

## FIG. 5

## FIG. 6

## FIG. 7

Difference of
probe data

## FIG. 8

pred. fat

lab. fat